**Europäisches Patentamt**

**European Patent Office**

**Office européen des brevets**

(11) Veröffentlichungsnummer: **0 391 151**
**A2**

(12) **EUROPÄISCHE PATENTANMELDUNG**

(21) Anmeldenummer: **90105412.2**

(22) Anmeldetag: **22.03.90**

(51) Int. Cl.5: **C12N 1/20**, //(C12N1/20, C12R1:13)

(30) Priorität: **03.04.89 DE 3910682**

(43) Veröffentlichungstag der Anmeldung:
**10.10.90 Patentblatt 90/41**

(84) Benannte Vertragsstaaten:
**AT BE CH DE DK ES FR GB IT LI LU NL SE**

(71) Anmelder: **BAYER AG**

**D-5090 Leverkusen 1 Bayerwerk(DE)**

(72) Erfinder: **Frommer, Werner, Prof.Dr.**
**Claudiusweg 17**
**D-5600 Wuppertal 1(DE)**
Erfinder: **Kanne, Reinhard, Dr.**
**Hüscheiderstrasse 80**
**D-5090 Leverkusen 3(DE)**
Erfinder: **Neupert, Manfred, Dr.**
**Drosselweg 10**
**D-5090 Leverkusen 3(DE)**
Erfinder: **Rast, Hans-Georg, Dr.**
**Rommerscheider Höhe 10**
**D-5060 Bergisch-Gladbach(DE)**
Erfinder: **Springer, Wolfgang, Dr.**
**Katernberger Schulweg 31**
**D-5600 Wuppertal(DE)**
Erfinder: **Tillmann, Werner, Dr.**
**Am Heidkamp 3**
**D-5090 Leverkusen 3(DE)**

(54) **Mikrobieller Abbau von mehrfach halogenierten Aromaten.**

(57) Bakterien der Gattung Brevibacterium, die Dibenzofuran und 2-Bromdibenzofuran als alleinige Kohlenstoff-quelle verwerten können, werden zur biologischen Reinigung Halogendibenzodioxin und -dibenzofuran haltiger Böden, Sedimente und Sickerwässer eingesetzt. Solche Mikroorganismenstämme werden zum mikrobiellen Abbau von mehrfach halogenierten polyzyklischen Aromaten, insbesondere von Halogendibenzo-p-dioxinen und Dibenzofuranen verwendet. Dabei können monozyklische Aromate, vorzugsweise Phenol oder Toluol, als Induktoren zugesetzt werden.

## Mikrobieller Abbau von mehrfach halogenierten Aromaten

Der mikrobielle Abbau von halogenierten Dibenzo-p-dioxinen wurde bisher nur wenig untersucht und beschränkt sich auf zwei Arbeiten von KLECKA und GIBSON mit Beijerinckia spec. (1) und BUMPUS et al. mit dem Weißfäulepilz Ph. chrysosporium (2). KLECKA und GIBSON fanden die Umsetzung von Dibenzodioxin, 1- und 2-Chlordibenzodioxin zu den entsprechenden Dihydrodiol-Derivaten (ohne Dechlorierung) durch eine Beijerinckia spec. nach Anzucht mit Succinat. Ein weitergehender Abbau mit Chloridfreisetzung wurde nicht beobachtet. BUMPUS et al. (2) beschrieben die Bildung von 27,9 p Mol $^{14}CO_2$ (~2 % der theoretisch freisetzbaren Menge) aus 1,25 nMol 2,3,7,8 TCDD nach 30 Tagen Inkubation mit dem Weißfäulepilz Phanerochaete chrysosporium bei Wachstum auf Glucose (56 mMol). Während über eine Anwendung der Abbaureaktion durch Beijerinckia spec. nichts bekannt ist, versuchen z.B. HÜTTERMANN und TROJANOWSKI (3) den Einsatz der Weißfäulepilze für die Bodendekontaminierung durch Verwendung von Stroh als C-Quelle.

Bei eigenen Untersuchungen des Abbaus von halogenierten Benzolen wurden andererseits Stämme gefunden, die mit hoher Rate Dibenzodioxin, Dibenzofuran und 2-Bromdibenzofuran oxidieren konnten, jedoch nicht zu einem vollständigen Abbau dieser Substanzen in der Lage waren. Es sollten daher Mikroorganismen angereichert und isoliert werden, die Dibenzofuran, Dibenzodioxin und 2-Bromdibenzofuran als Kohlenstoffquelle nutzen können, um zu Stämmen zu gelangen, die auch halogenierte Dibenzodioxine effektiv abbauen können.

Diese Aufgabe konnte erfindungsgemäß dadurch gelöst werden, daß aus Rheinwasserproben neue Mikroorganismen-Stämme mit den Bezeichnungen RST 69-211, RST 69-233 und RST 69-1361 der Gattung Brevibakterium angereichert und isoliert wurden. Diese Stämme wurden bei der Deutschen Sammlung von Mikroorganismen (DSM), Grisebachstraße 8, D-3400 Göttingen, Bundesrepublik Deutschland, in Übereinstimmung mit den Bestimmungen des Budapester Vertrages über die internationale Anerkennung der Hinterlegung von Mikroorganismen für die Zwecke von Patentverfahren am 20.07.88 mit den Hinterlegungsnummern DSM 4714, 4715 und 4716 hinterlegt.

Die vorliegende Erfindung erstreckt sich auch auf die Mutanten und Varianten dieser Stämme, welche die für die Ausführung der Erfindung wesentlichen Merkmale und Eigenschaften aufweisen.

Diese neuen Stämme werden erfindungsgemäß zum mikrobiellen Abbau von mehrfach halogenierten polyzyklischen Aromaten, insbesondere von Halogendibenzo-p-dioxinen und Dibenzofuranen verwendet. Dabei können monozyklische Aromate, vorzugsweise Phenol oder Toluol als Induktoren zugesetzt werden.

Hauptanwendungsgebiete für die neuen Stämme sind die mikrobiologische Abwasserreinigung und Bodendekontaminierung. Die zuletzt genannte Anwendung ist im Hinblick auf die Entsorgung und Wiederaufarbeitung von Altdeponien, die mit halogenierten polyzyklischen Aromaten kontaminiert sind, von großer Bedeutung.

Im folgenden wird die Erfindung anhand von Ausführungsbeispielen näher erläutert.


## Wachstumsbedingungen und Charakterisierung der Reinkulturen

Bakterien, die Dibenzo-p-dioxin und Dibenzofuran als alleinige C-Quelle verwerten konnten, wurden aus Rheinwasser isoliert. Hierzu wurde Rheinwasser mit Mineralsalzen versetzt, Dibenzo-p-dioxin oder Dibenzofuran (0,5 g/l) als alleinige C-Quelle zugesetzt und mehrere Wochen unter Schütteln bei 250 UpM in 250 ml Portionen in 1l-Erlenmeyerkolben bei 28 °C inkubiert. Nach Entstehen einer Gelb- bzw. Braunfärbung wurden die Kolben auf frisches Mineralsalzmedium mit Dibenzofuran überimpft und abschließend über Verdünnungsreihen auf sterilen Agarmedien die Bakterien als Reinkulturen isoliert. Hierfür wurde das Mineralsalzmedium mit Hefeextrakt (0,5 g/l) ergänzt, Dibenzo-p-dioxin bzw. Dibenzofuran (0,5 g/l) als 10 %ige DMSO-Lösung zugesetzt und zur gleichmäßigen Verteilung des unlöslichen Dibenzofurans zusätzlich Tween 80 (50 mg/l) zugegeben. Dibenzofuran und Dibenzo-p-dioxin abbauende Bakterien konnten an der Verfärbung des Mediums (gelb bzw. braun) erkannt werden.

Isolierte Reinkulturen wurden auf Agarplatten mit Komplexmedium (Standard I, Fa. Merck) gehalten. Langzeitkonserven wurden in Flüssigstickstoff aufbewahrt.

Es gelang, aus Rheinwasser 10 verschiedene Reinkulturen anzureichern und zu isolieren, die Dibenzofuran und Dibenzo-p-dioxin als C-Quelle verwerten konnten (Tab. 1). Ein Test unter den gleichen Bedingungen zeigte, daß nur die gram-positiven Isolate in der Lage waren, 2-Bromdibenzofuran abzubauen. Für die weiteren Untersuchungen wurden daher diese gram-positiven Isolate eingesetzt, da hier ein Abbau der Halogendibenzo-p-dioxine am ehesten zu erwarten war.

Eine nähere Charakterisierung der Isolate RST 69-211, RST 69-233 und RST 69-1361 lieferte folgende Resultate: Gram-positive, unbewegliche kokkoide Stäbchen mit einem deutlichen Stäbchen-Kokkus-Zyklus in der frühen Wachstumsphase. Katalase war positiv, Oxidase und KOH-Test waren negativ. Zellwandpräparationen enthielten direkt verknüpfte m-DAP; Mycolsäuren und N-Glycolylester waren nicht vorhanden. Als C-Quellen wurden von allen Stämmen verwertet (Tab. 2):

Glucose, Saccharose, Glycerin, Brenztraubensäure, Essigsäure, Benzoesäure und Salicylsäure, jedoch nicht Mannit. Diese Ergebnisse lassen eine eindeutige Zuordnung zum Genus Brevibacterium zu. Eindeutige Unterschiede traten hingegen zu jedem der vier in Bergey's Manual of Systematic Bacteriology beschriebenen Spezies auf. Eine weitere Bestimmung der Spezies war daher nicht möglich. Die Stämme wurden am 20.07.88 mit den Hinterlegungsnummern DSM 4714, 4715 und 4716 (für RST 69-211, RST 69-233 und RST 69-1361) als Patentstämme hinterlegt.

Unterschiede zwischen den drei Stämmen waren bei der Verwertung von Phenol, Toluol und Milchsäure zu erkennen. Anilin und Chlorbenzol konnten in der eingesetzten Konzentration nicht als C-Quelle verwertet werden. Das Entstehen einer Braun- bzw. Orangefärbung deutete jedoch auf einen unvollständigen Abbau dieser Verbindungen hin. Stamm 233 war darüber hinaus in der Lage, mit Benzol und O-Xylol als C-Quelle zu wachsen und 4-Chlorphenol sowie 5-Chlorsalicylsäure abzubauen. Wegen des breiten Abbauspektrums, insbesondere auch der monozyklischen Halogenaromaten in Stamm 233 wurde der weitere Abbau der Dioxine mit diesem Stamm untersucht.

Abbauuntersuchungen

Abbauuntersuchungen mit polyzyklischen Aromaten wurden in 1l-Erlenmeyerkolben mit teflonbeschichteten Schraubverschlüssen durchgeführt, die 20 ml mineralisches Medium mit den entsprechenden Zusätzen enthielten. Für die Rückstandsanalysen wurde der gesamte Kolbeninhalt aufgearbeitet. Nur so waren reproduzierbare Messungen auch in geringen Konzentrationen durchführbar.

Die Anzucht größerer Zellmengen erfolgte in einem 10 l BE-Fermenter (Fa. Braun, Melsungen). Als Anzuchtsmedium wurde Standard I mit Phenol (0,8 g/l) bzw. mineralisches Medium mit Hefeextrakt (0,5 g/l) verwendet. Im letzteren Fall wurden weitere C-Quellen als DMSO-Lösungen (2 bis 10 %ig, sterilfiltriert), über einen separaten, mit Lösungsmittel gesättigten Zuluftstrom (Toluol, Benzol, O-Xylol) oder als konzentrierte wäßrige Lösungen (Phenol, Salicylsäure; 20 g/l) zudosiert. Die Fermentationsbedingungen waren: 800 UpM, 28° C, 1 l Luft/min.

Die Rückstandsanalyse der aromatischen Verbindungen wurde mit HPLC durchgeführt. Hierzu wurde der gesamte Inhalt eines 1l-Erlenmeyerkolbens (20 ml) mit 2 ml einer 30 %igen Brij 58-Lösung (in DMSO) 30 Minuten bei 28° C geschüttelt, anschließend 20 ml Dimethylformamid zugegeben, erneut 30 Minuten geschüttelt und in 50 ml Falcon Zentrifugenröhrchen gefüllt. Nach 15 Minuten Zentrifugation bei 4.000 g wurde im Überstand die Konzentration der zu untersuchenden Verbindungen gemessen. Als Kontrollen dienten Ansätze, die mit HCl auf pH 2 gestellt wurden. Die Auftrennung der Überstände wurde mit einer RP8-Säule durchgeführt, als Laufmittel dienten Wasser/Acetonitril-Gemische mit unterschiedlichen, an der Polarität der Aromaten orientierten, Zusammensetzungen (8:2 bis 2:8). Die Detektion wurde mit einem UV-Detektor (Shimadzu) mit variabler Wellenlänge jeweils beim UV-Maximum der zu untersuchenden Verbindungen durchgeführt. Diese Art der Rückstandsanalyse lieferte bessere Wiederfindungsraten ( > 90 %) als die wesentlich aufwendigeren Extraktionsmethoden. Zur weiteren Verbesserung der Ergebnisse (geringere Streuung) konnte 1,4-Diaminoanthrachinon als interner Standard mit dem Dimethylformamid zugegeben werden. Für die HPLC-Analyse mußten in diesem Fall die Überstände mit 6N NaOH alkalischen gestellt werden. Die Nachweisgrenze der HPLC-Analytik lag zwischen 10 und 100 $\mu$g/l.

Für die Bestimmung der Chlorid- und Bromidbilanzen wurde ein chloridfreies rein mineralisches Medium mit weniger Phosphat (10 mM) verwendet. Zur Bestimmung der Halogenidionen Konzentration wurden die Proben mit $H_2SO_4$ auf pH 2 gestellt, abzentrifugiert, sterilfiltriert und bis zur Analyse bei 4° C aufbewahrt. Die Konzentrationsbestimmung wurde mit einem Dionex-Ionenchromatographen 2000 i/SP mit einer HPIC AS4A-Trennsäule und HPIC AG4A-Vorsäule durchgeführt. Für die Bromidbestimmung wurde als Laufmittel 1,7 mM $Na_2CO_3$/1,8 mM $NaHCO_3$ verwendet. Für die Chloridbestimmung mußte mit 2 mM NaOH zunächst Chlorid eluiert werden, bevor mit $Na_2CO_3$/$NaHCO_3$ die anderen Ionen eluiert wurden. Auf diese Weise konnte für Chlorid problemlos eine Basislinientrennung erreicht werden. Die Nachweisgrenze der Methode lag bei 0,1 $\mu$M, d.h., die Proben konnten für die Ionenchromatographie 1:10 verdünnt werden.

Für die Anreicherung und Isolierung von Abbauprodukten der verschiedenen Dibenzo-p-dioxine wurden die Substanzen in DMSO/Tween 80 = 9/1 in möglichst hoher Konzentration (2 bis 10 %ig) gelöst, unter Rühren in 450 ml 0,1 M Phosphatpuffer, pH 7,2, in einem 1l-Erlenmeyerkolben bis zu einer Endkonzentration von 40 mg/l zudosiert und 50 ml einer Zellsuspension (40 g abzentrifugierte Zellmasse in 120 ml 0,1 M Phosphatpuffer, pH 7,2) aktiver Zellen zugefügt. Der Erlenmeyerkolben wurde bei 28°C mit 250 Upm geschüttelt, stündlich ein Aliquot entnommen und die Konzentration der Ausgangsverbindung bestimmt. Nach möglichst vollständigem Umsatz der Ausgangsverbindungen wurde der gesamte Ansatz mit n-Hexan oder Dichlormethan mehrfach extrahiert, der Extrakt getrocknet, eingeengt und auf präparativen DC-Platten (Fa. Merck) in Toluol-Dioxan-Essigsäure = 90-25-5 chromatographisch aufgereinigt. Banden, die beim Besprühen mit Folin-Reagenz eine Blaufärbung zeigten, wurden abgekratzt, eluiert und die Struktur über GC-MS und NMR weiter untersucht.

Meßbedingungen der $^1$H-NMR-Untersuchung

Die Messung wurde an einem 360-MHZ-Supercon-FT-NMR-Spektrometer Bruker AM 360 durchgeführt. Für die Messung wurde die chromatographisch isolierte, getrocknete Probe in $C_6D_6$ gelöst. Es wurden 32 Scans akkumuliert. Die chemische Verschiebung wurde auf den Inneren Standard Tetramethylsilan (0 ppm) bezogen. Die Auswertung erfolgte am gedehnten Spektrum (10 Hz/cm) anhand der aromatischen Protonensignale im Bereich 6 bis 7 ppm.

Massenspektrometrie

Die massenspektrometrische Untersuchung erfolgte durch GC-MS-Kopplung an einem Finnigan-MAT 8230 Massenspektrometer. Der vorgeschaltete Gaschromatograph war vom Typ Varian 3700. Die Gaschromatographie erfolgte mit Split 1:130, Einspritzblock 250°C, und dem Temperaturprogramm: 3 Minuten isotherm 70°C, Gradient von 70 auf 320°C mit 15°C/Minute an einer SE 30 Kapillare (20 m) bei einem Einspritzvolumen von 1 μl. Der Einlaß ins Massenspektrometer erfolgt durch direkte Kopplung. Die massenspektrometrischen Meßbedingungen waren Ionenstoßanregung von 70 eV und 3 kV Beschleunigungsspannung. Die Genauigkeit war +/- 0,2 amu im Bereich bis 1.000 amu.

Messung der Veratmungsraten

Zur Messung der Veratmungsraten wurden frisch angezüchtete oder eingefrorene Zellen in einer Konzentration von 0,2 g TS pro 10 ml in 0,1 M Phosphatpuffer, pH 7,2, suspendiert. 1 ml dieser Zellsuspension wurde in ein 100 ml temperierbares, verschließbares und rührbares Inkubationsgefäß mit einer Orion $O_2$-Elektrode pipettiert, die Grundatmung über 5 Minuten registriert, 100 μl der Testsubstratlösung (10 mg/ml in DMSO) zugegeben und weitere 5 Minuten die $O_2$-Aufnahme gemessen. Aus der Differenz der beiden Veratmungsraten konnte die spezifische $O_2$-Aufnahmerate mit den aromatischen Testsubstraten errechnet werden.

Abbau von 2-Bromdibenzofuran und halogenierter Dibenzo-p-dioxine durch Stamm RST 69-233

Stamm 233 konnte 2-Bromdibenzofuran (80 μM) bei einer Zelldichte von 2 g TS/l innerhalb von 24 Stunden auf weniger als 10 μg/l (= 40 nM) abbauen. Die zeitlich verzögerte Freisetzung von anorganischem Bromid deutete auf eine vorübergehende Anreicherung von Zwischenpro dukten hin. Nach 72 Stunden wurden ca. 75 % des eingesetzten organisch gebundenen Broms als freies Bromid wiedergefunden. Die Art der Zellzucht hatte nur einen geringen Einfluß auf den Abbau von 2-Bromdibenzofuran.

Ein völlig anderes Ergebnis war beim Abbau von 2,3-Dichlordibenzo-p-dioxin zu beobachten. Während nicht induzierte Zellen innerhalb von 48 Stunden nur einen teilweisen Abbau von 2,3-Dichlordibenzo-p-dioxin zeigten, war bei Phenol-induzierten Zellen bereits nach 6 Stunden ein vollständiges Verschwinden der Ausgangssubstanz zu erkennen. Die zeitlich verzögerte Freisetzung von Chlorid war hier jedoch noch deutlicher zu sehen. Erst bei der Messung nach 24 Stunden war Chlorid im Inkubationsansatz nachweisbar, dessen Konzentration nach 120 Stunden über 130 μM erreichte. Dies entsprach einer nahezu quantitativen Freisetzung des organisch gebundenen Chlors zu Chlorid. Offensichtlich benötigte Stamm 233 einen zusätzlichen Induktor, z.B. Phenol, für die Induktion der Halogendibenzo-p-dioxin abbauenden Enzyme. In einem weiteren Ansatz wurde daher der Abbau verschiedener Halogendibenzo-p-dioxine und von 2-Bromdibenzofuran mit Phenol-induzierten Zellen im Vergleich gemessen. Während die in einem Ring

4

halogenierten Substrate innerhalb von 6 Stunden bis zur Nachweisgrenze abgebaut wurden, war bei 2,7-Dichlordibenzo-p-dioxin ein vollständiges Verschwinden der Ausgangsverbindung erst nach 30 Stunden zu erkennen. Bei allen halogenierten Derivaten konnte eine nahezu quantitative Halogenidfreisetzung (Tab. 3) gemessen werden.

## Induktion des Halogendibenzo-p-dioxinabbaus in Stamm RST 69-233

Zur Klärung der Induktion des Dibenzo-p-dioxinabbaus wurde Stamm 233 mit verschiedenen aromatischen Verbindungen angezüchtet und die Veratmung der unterschiedlichen mono- und polyzyklischen Aromaten gemessen. Wie von den Abbauergebnissen her zu erwarten, wurde die Dibenzodioxin-Oxygenase-Aktivität durch 2-Bromdibenzofuran oder 2,3-Dichlordibenzo-p-dioxin alleine nur relativ schwach oder nicht induziert (Tabe. 4). Verschiedene monozyklische Aromaten (Phenol, Toluol) wirkten als gute Induktoren der Dibenzodioxin-Oxygenase. Benzol und Salicylsäure als Wachstumssubstrate zeigten keinen Induktionseffekt.

Bei Verwendung von Phenol als zusätzliche C-Quelle war ein deutlicher Unterschied zur Anzucht mit Phenol als alleinige C-Quelle zu erkennen. In allen Fällen wurden zusätzlich Phenolhydroxylase- und Salicylsäure-Oxygenase-Aktivitäten gemessen. Das Ergebnis mit Benzol gewachsenen Zellen deutet auf verschiedene Enzyme für die Oxidation von mono- und polyzyklischen Aromaten hin, da hier bei sehr hohen Umsatzraten mit Toluol keinerlei Oxidation von Dibenzo-p-dioxin gemessen wurde. Bei allen Anzuchten mit Aromaten wurden hohe Ringspaltungsaktivitäten mit Brenzcatechin gefunden.

In einem weiteren Test wurde die Substratspezifität des Oxidationssystems nach Wachstum mit einem guten Induktor (Toluol) untersucht (Tab. 5). Von den polyzyklischen Aromaten wurde Phenoxazin mit der höchsten Rate oxidiert, während Dibenzofuran, Dibenzodioxin, Phenothiazin, Naphthalin und Inden mit deutlich geringerer Rate oxidiert wurden. Zusätzliche Halogensubstituenten verlangsamten die Umsatzraten ebenfalls. Kein $O_2$-Verbrauch konnte mit Xanthon, Carbazol, Phenazin, Thioxanthon, Anthrachinon und 2,7-Dichlordibenzo-p-dioxin gemessen werden. Hier lagen die Oxidationsraten zum Teil unterhalb der Meßgrenze, da bei längerer Inkubationszeit ein langsamer Abbau z.B., von 2,7-Dichlordibenzo-p-dioxin gemessen wurde. Ein weiterer Hinweis auf einen langsamen Abbau war das Auftreten einer Verfärbung ($E_{max}$ = 510 nm) bei längerer Inkubation mit Thioxanthon. Ebenfalls stark gefärbte Abbauprodukte entstanden bei der Oxidation von Phenoxazin ($E_{max}$ = 571 nm) und Phenothiazin ($E_{max}$ = 591 nm).

Die hohen Umsatzraten von 4-Chlorbrenzcatechin und die Oxidation von 3,5-Dichlorbrenzcatechin, 5-Chlorsalicylsäure, 4-Chlorphenol und Chlorbenzol stehen in Einklang mit den in den Abbauuntersuchungen gefundenen Halogenidfreisetzungen aus 2-Bromdibenzofuran und den Dichlordibenzo-p-dioxinen. Offensichtlich besitzt Stamm 233 die Enzyme für den vollständigen Abbau sowohl von monozyklischen als auch von polyzyklischen Halogenaromaten.

## Charakterisierung von Abbauprodukten

Beim Abbau von 2-Bromdibenzo-p-dioxin, 2,3-Dichlor- und 2,7-Dichlordibenzo-p-dioxin traten in den ersten Stunden nach Zugabe Zwischenprodukte auf, die nach längerer Inkubationszeit wieder verschwanden. Wurden Ansätze zu einem frühen Zeitpunkt (3 bis 6 Stunden) extrahiert, so ließen sich aus diesen Extrakten Abbauprodukte chromatographisch reinigen, die beim Ansprühen mit Folin-Reagenz die typische Blaufärbung aromatischer Hydroxyverbindungen zeigten. Die Aufnahme von Massenspektren über GC-MS ergab bei allen Abbauprodukten eine Zunahme der Molmasse um 16, d.h., Monohydroxyderivate der Ausgangsverbindungen (Tab. 6). Anhand des Zerfallsmusters ließ sich jedoch keine Aussage über die Hydroxylierungsstelle machen. Um den Substitutionsort der Hydroxygruppe zu ermitteln, wurden zum Vergleich Hochfeld-Protonen-NMR-Spektren von 2-Bromdibenzodioxin, 2,7-Dibromdibenzodioxin und vom unsubstituierten Dibenzodioxin gemessen. Das unsubstituierte Dibenzodioxin ergibt erwartungsgemäß ein $A_2$-$B_2$-Spektrum mit engem Bereich der chemischen Verschiebung, das 2,7-Dichlorderivat aus Symmetriegründen ein aromatisches 1,2,4-Protonensystem. Das 2-Bromderivat zeigt den $A_2B_2$-Teil und das 1,2,4-Substitutionsmuster des bromierten Rings. Der $A_2B_2$-Teil des Spektrums fehlt beim hydroxylierten 2-Bromdibenzodioxin. Daraus ist zu schließen, daß die Hydroxylierung im unsubstituierten Benzolring erfolgt ist. Das Spektrum ist zu deuten als Gemisch von 2-Brom-7-hydroxydibenzodioxin und 2-Brom-8-hydroxydibenzodioxin (4 verschiedene aromatische 1,2,4-Protonengruppen). Entscheidend für die Separierung der Protonensignale war beim hydroxylierten Produkt die Verwendung von Hexadeuterobenzol als NMR-Lösungsmittel.

5

Tabelle 1

| Abbaueigenschaften und Gramverhalten verschiedener Dibenzofuran und Dibenzo-p-dioxin abbauender Mikroorganismen (Rheinwasserisolate). | | | | |
|---|---|---|---|---|
| Stamm | Gramfärbung | Abbau von Dibenzofuran | Abbau von Dibenzo-p-dioxin | Abbau von 2-Bromdibenzofuran |
| 69-7 | - | - | + | - |
| 69-8 | - | - | + | - |
| 69-41 | - | - | + | - |
| 69-42 | - | + | + | - |
| 69-112 | - | - | + | - |
| 69-234 | - | - | + | - |
| 69-2351 | - | - | + | - |
| 69-211 | + | + | + | + |
| 69-233 | + | + | - | + |
| 69-1361 | + | + | + | + |

Tabelle 2

| Biochemische Eigenschaften der 2-Bromdibenzofuran abbauenden Stämme RST 69-211, -233 und -1361. Für den Wachstumstest mit flüchtigen Aromaten wurden die Substrate über die Dampfphase zudosiert. | | | |
|---|---|---|---|
| Eigenschaft | 69-211 | 69-233 | 69-1361 |
| Gram-Verhalten | + | + | + |
| Oxidase | - | - | - |
| Katalase | + | + | + |
| Wachstum mit C-Quelle (g/l): | | | |
| Glucose (10) | + | + | + |
| Saccharose (10) | + | + | + |
| Mannit (1) | - | - | - |
| Glycerin (2) | + | + | + |
| Brenztraubensäure (2) | + | + | + |
| Milchsäure (2) | + | - | + |
| Essigsäure (2) | + | + | + |
| Benzoesäure (2) | + | + | + |
| Salicylsäure (2) | + | + | + |
| Phenol (0,8) | - | + | - |
| Anilin (0,8) | - | - | - |
| Benzol | n.g. | + | n.g. |
| Toluol | - | + | + |
| o-Xylol | n.g. | + | n.g. |
| Chlorbenzol | - | - | - |
| Abbau von (g/l): | | | |
| 5-Chlorsalicylsäure (0,1) | n.g. | + | n.g. |
| 4-Chlorphenol (0,1) | n.g. | + | n.g. |

Tabelle 3

| Halogenidbilanz beim Abbau von 2-Bromdibenzofuran und verschiedenen Halogendibenzo-p-dioxinen durch Phenolinduzierte 69-233-Zellen nach 5 bzw. 10 Tagen. Die Halogenidkonzentration in einer Kontrolle ohne Zusatz von Halogenaromaten betrug < $\mu$M. | | | |
|---|---|---|---|
| Testsubstrat | Eingesetzte Substratkonzentration ($\mu$M) | Substratendkonzentration ($\mu$M) | Halogenidendkonzentration ($\mu$M) |
| 2-Bromdibenzofuran | 73 | < 0,05 | 60 |
| 2-Bromdibenzo-p-dioxin | 68 | < 0,05 | 63 |
| 2,3-Dichlordibenzo-p-dioxin | 61 | < 0,1 | 129 |
| 2,7-Dichlordibenzo-p-dioxin | 30 | < 0,1 | 56 |

Tabelle 4

Induktion der Dibenzodioxin-Oxygenase und anderer Oxygenasen in Abhängigkeit von den Anzuchtsbedingungen bei Stamm 69-233. Gemessen wurde die spezifische $O_2$-Aufnahmerate (nMol/min xmg TS). N.g. = nicht gemessen; $0 \equiv< 0,5$ nMol/min xmg TS.

| Anzuchtsbedingungen / Testsubstrat | Phenol als C-Quelle | Phenol als Induktor | Toluol | Benzol | Salicylsäure | 2-Bromdibenzofuran | Phenol + 2,3 DCDD | 2,3-DCDD als Induktor | Standard I |
|---|---|---|---|---|---|---|---|---|---|
| Dibenzodioxin | 0 | 2,5 | 3,8 | 0 | 0 | 1,0 | 3,8 | 0 | 0 |
| Salicylsäure | 20,8 | n.g. | 22,2 | 23,2 | 20,8 | 20,8 | n.g. | n.g. | 0 |
| Phenol | 67,7 | 12,2 | 62,5 | 187,5 | n.g. | 78,2 | 70,3 | 0 | 0 |
| Toluol | 4,7 | 46,8 | 52,1 | 99,0 | n.g. | 52,2 | 26,0 | 0 | 0 |
| Brenzcatechin | 59,8 | n.g. | 312,5 | 296,7 | 230,0 | 220,0 | 172,0 | n.g. | 0 |

Tabelle 5

| Spezifische $O_2$-Aufnahmeraten mit verschiedenen mono- und polyzyklischen Aromaten durch Stamm 69-233 nach Wachstum mit Toluol. $0 \equiv < 0,5$ nMol/min x mg TS. | |
| --- | --- |
| Substrat | Spezifische $O_2$-Aufnahmerate (nMol/min x mg TS) |
| Dibenzo-p-dioxin | 3,8 |
| 2-Bromdibenzo-p-dioxin | 3,3 |
| 2,3-Dichlordibenzo-p-dioxin | 0,6 |
| 2,7-Dichlordibenzo-p-dioxin | 0 |
| Dibenzofuran | 5,2 |
| 2-Bromdibenzofuran | 3,3 |
| Phenoxazin | 22,2 |
| Phenazin | 0 |
| Carbazol | 0 |
| Phenothiazin | 3,3 |
| Thioxanthon | 0 |
| Xanthen | 6,6 |
| Xanthon | 0 |
| Anthrachinon | 0 |
| Naphthalin | 4,5 |
| Inden | 7,8 |
| Benzol | 16,9 |
| Toluol | 52,1 |
| Chlorbenzol | 1,6 |
| Phenol | 62,5 |
| Chlorphenol | 16,3 |
| Salicylsäure | 22,2 |
| 5-Chlorsalicylsäure | 1,2 |
| Benzoesäure | 0 |
| Brenzcatechin | 312,5 |
| 4-Chlorbrenzcatechin | 160,2 |
| 3,5-Dichlorbrenzcatechin | 16,9 |

Tabelle 6

| GC-MS-Daten von 2-Bromdibenzo-p-dioxin, 2,3-Dichlor-, 2,7-Dichlordibenzo-p-dioxin und deren Abbauprodukte durch Stamm 69-233. | | |
|---|---|---|
| Verbindung | m/L (rel. Intensität) | Zerfallsprodukte |
| 2-Bromdibenzo-p-dioxin | 262 (100) | Molekülion |
| | 183 ( 15) | -Br |
| | 155 ( 40) | -CO |
| | 125 ( 30) | -CO |
| Abbauprodukt von 2-Bromdibenzo-p-dioxin | 278 (100) | Molekülion |
| | 199 ( 12) | -Br |
| | 171 ( 20) | -CO |
| | 143 ( 5) | -CO |
| | 115 ( 20) | -CO |
| 2,3-Dichlordibenzo-p-dioxin | 252 (100) | Molkülion |
| | 217 ( 5) | -Cl |
| | 189 ( 50) | -CO |
| | 161 ( 20) | -CO |
| | 126 ( 90) | -Cl |
| Abbauprodukt von 2,3-Dichlordibenzo-p-dioxin | 268 (100) | Molekülion |
| | 233 ( 5) | -Cl |
| | 205 ( 15) | -CO |
| | 177 ( 3) | -CO |
| | 149 ( 12) | -CO |
| | 114 ( 3) | -Cl |
| 2,7-Dichlordibenzo-p-dioxin | 252 (100) | Molekülion |
| | 217 | -Cl |
| | 189 | -CO |
| | 161 | -CO |
| Abbauprodukt von 2,7-Dichlordibenzo-p-dioxin | 268 (100) | Molekülion |
| | 233 ( 15) | -Cl |
| | 205 ( 30) | -CO |
| | 177 ( 2) | -CO |
| | 149 ( 12) | -CO |

Literatur

1) G.M. Klecka, D.T. Gibson: Metabolism of dibenzo-p-dioxin and chlorinated dibenzo-p-dioxins by a Beijerinckia species. Appl. Environ Microb. 39 (1980), 288

2) J.A. Bumpus, M.T.D. Wright, S.D. Aust: Oxidation of persistent environmental pollutants by a white rot fungus Science, 228 (1985), 1434

3) A. Hüttermann, J. Trojanowski: Ein Konzept für eine in-situ Sanierung von mit schwer abbaubaren Aromaten belasteten Böden durch Inkubation mit dafür geeigneten Weißfäulepilzen und Stroh.
V. Franzius (Hrsg.): Sanierung kontaminierter Standorte 1986 "Neue Verfahren zur Bodenreinigung" Abfallwirtschaft in Forschung und Praxis, Band 18, 205-218, Berlin, E. Schmidt Verlag (1987)

4) W. Springer, H.G. Rast: Biologischer Abbau mehrfach halogenierter mono- und polyzyklischer Aromaten. GWF Wasser/Abwasser 129 (1988), 70.

**Ansprüche**

1. Bakterien der Gattung Brevibacterium, die Dibenzofuran und 2-Bromdibenzofuran als alleinige C-Quelle verwerten können, zur biologischen Reinigung Halogendibenzodioxin und -dibenzofuran haltiger Böden, Sedimente und Sickerwässer.

2. Mikroorganismenstämme RST 69-211, RST 69-233 und RST 69-1361 nach Anspruch 1, einschließlich ihrer Mutanten und Varianten für die mikrobiologische Abwasserreinigung und Bodendekontaminierung.

3. Verwendung der Stämme nach Anspruch 2 zum mikrobiellen Abbau von mehrfach halogenierten polyzyklischen Aromaten.

4. Verwendung gemäß Anspruch 3, zum Abbau von Halogendibenzo-p-dioxinen und Dibenzofuranen.

5. Verwendung gemäß Anspruch 4 unter Zusatz von monozyklischen Aromaten, vorzugsweise Phenol oder Toluol, als Induktoren.